# EUROPEAN PATENT SPECIFICATION

(11) **EP 3 039 064 B1**
(45) Date of publication and mention of the grant of the patent: **24.03.2021**
(21) Application number: 14750228.0
(22) Date of filing: 11.08.2014
(51) Int. Cl.: C08J 11/14, C08J 11/16, C11B 13/00, A61K 36/81, C07C 67/48, C08G 63/06

(54) **EXTRACTION METHOD OF A POLYESTER POLYMER OR CUTIN FROM THE WASTED TOMATO PEELS AND POLYESTER POLYMER SO EXTRACTED**
VERFAHREN ZUR EXTRAKTION EINES POLYESTERPOLYMERS ODER VON CUTIN AUS TOMATENSCHALENABFÄLLEN UND AUF DIESE WEISE EXTRAHIERTES POLYESTERPOLYMER
PROCÉDÉ D'EXTRACTION D'UN POLYMÈRE DE POLYESTER OU DE CUTINE À PARTIR D'ÉPLUCHURES DE TOMATES ET POLYMÈRE DE POLYESTER AINSI EXTRAIT

(30) Priority: 26.08.2013 IT PR20130066
(43) Date of publication of application: 06.07.2016
(73) Proprietor: CHIESA VIRGINIO E C. SOCIETA' SEMPLICE AGRICOLA, 46013 Canneto Sull'Oglio (MN) (IT)
(72) Inventor: CIGOGNINI, Ilaria, I-43122 Parma (IT); MONTANARI, Angela, I-42049 Sant'Ilario d'Enza (RE) (IT); DE LA TORRE CARRERAS, Rosa, E-06490 Badajoz (ES); CARDOSO BERNET MONTSERRAT, Gomez, E-06490 Badajoz (ES)
(74) Representative: Fanzini, Valeriano
(86) International application number: PCT/EP2014/067187
(87) International publication number: WO 2015/028299

(56) References cited:
- WO-A1-2010/093320
- US-A- 4 732 708
- US-A1- 2002 043 577
- US-A1- 2011 319 504
- STANLEY F. OSMAN ET AL: "Method for the Production and Characterization of Tomato Cutin Oligomers", JOURNAL OF AGRICULTURAL AND FOOD CHEMISTRY, vol. 43, no. 8, 1 August 1995 (1995-08-01) , pages 2134-2137, XP055120098, ISSN: 0021-8561, DOI: 10.1021/jf00056a033

## Description

### FIELD OF THE INVENTION

The invention relates to the development of a method for the valorization of industrial processing tomato by-products (skins), which consists in the extraction of a polyester polymer composed of a complex mixture of interesterified, long-chain 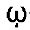 -hydroxy acid with typically a 16- or 18-carbon skeleton, named cutin, from the waste of tomato peels.

In particular the invention relates to a method for producing monomers and oligomers of tomato cutin, extracted from tomato peels, whose main component is 10,16-dihydroxyhexadecanoic acid.

### STATE OF THE ART

Cutin is a support biopolyester involved in waterproofing the leaves and fruits of higher plants. Cutin is the main component (between 40% and 85%, w/w) of the plant cuticle, the continuous and lipidic extracellular membrane that covers the aerial parts of leaves, fruits and non-lignified stems of plant. Associated to cutin there are cuticular waxes or lipids soluble, they are embedded within the matrix cuticular waxes intracuticulares, or deposited on the outer surface of the cuticle waxes epicuticular. Furthermore, the cuticle also contains a number of components lipid such as polysaccharides (mainly cellulose and pectin), polypeptides and phenolic compounds. Thus, the plant cuticle can be considered a polyester waxes complex associates, very small hydrophobic nature reactivity, since most of the carboxylic groups present in the membrane are esterified with aliphatic hydroxyl groups of other fatty acids, which covers the aerial part of leaves, fruits and non-lignified stems of plant. Considering the average weight of an isolated cuticle, cutin can be considered the major lipid plant polymer (between 40 and 85% w/w of the plant cuticle).

Cutin plays an important role in cuticle as a structural component and as a defense barrier toward pathogens and the uncontrolled loss of water, as well as in transporting substances across the plant tissues.

From a chemical point of view, cutin is defined as a polymeric network of polyhydroxylated C16 and C18 fatty acids cross-linked by ester bonds, as it is reported in A. Heredia, "Biophysical and biochemical characteristics of cutin, a plant barrier biopolymer", Biochimica et Biophysica Acta 1620 (2003) 1-7. Most of them are exclusively fatty acids C16 belonging to the family in which the 10,16-dihidroxihexadecanoic acid, and its positional isomer-9,16-dihidroxihexadecanoic acid, are the main components for tomato cutin. Only a small fraction of the cutin are formed by fatty acids belonging to the family C18, including acids 9,10-epoxy-18-hydroxyoctadecanoic and 9,10,18-trihidroxioctadecanoic are the most abundant, although some derivatives may be present as unsaturated cutin, as reported in G. M. Lopez "Biomecanica de la epidermis y la cutìcola del fruto de tomate (solanum lycopersicum I.) y su la relacion con el agrietado" (2006), Ph. Thesis, facultad de Ciencias, Departamento de Biologia molecular y Bioquimica, Universidad de Malaga.

Cutin is generally extracted from plant material with methods using enzymatic treatments, organic solvents or acid hydrolysis, as reported in literature.

All the methods reported in literature are aimed to analyse the composition of cutin and they show only studies and characterizations of cutin, but not how to use cutin as raw materials for other preparations, that is the target of this invention.

In particular in the enzymatic method the cuticular membrane is isolated by enzymatic treatment to degrade the polysaccharides. The cuticle is then extracted (solid-liquid extraction by Soxhlet with organic solvent CHCl3 and MeOH) to remove soluble material, waxes and other small molecules, yielding cutin enriched residues, as reported in R. Jarvinen, A. J.D. Silvestre, A. M. Gil, H. Kallio, "Solid state 13CP-MAS NMR and FT-IR spectroscopic analysis of cuticolar fractions of berries and suberized membranes of potato" Journal of Food Composition and Analysis 24 (2011) 334-345 and in H. Kallio, R. Nieminen, S. Tuomasjukka, M. Hakala "Cutin composition of Five Finnish berries" Journal of Agricultural and Food Chemistry 54 (2006), 457-462.

With this method, it's possible to obtain only dewaxed peels and peels without sugars, but not cutin. In this method lacks a procedure that permits to isolate from dewaxed peels cutin. Moreover this method is solvent consuming, considering that the Soxhlet extraction utilizes organic toxic solvent as chloroform (chloroform is carcinogen) and methanol and this extraction has to be repeated to increase the yield of extraction. So the procedure doesn't appear very eco-friendly.

Another organic solvent used for cutin extraction is aceton, as reported in the U.S.A. Patent n. US 2011/0319504 A1 "Method for the manufacture of oligo- and polyesters from a mixture of carboxylic acid obtained from suberin and/or cutin and use thereof", where cutin is isolated by a solvent extraction with aceton in a Soxhlet apparatus and then with a system of refluxing in a basic solution.

This method has been experimented, but the final extract didn't present the usual aspect of cutin. In fact the IR spectrum on this sample didn't show the usual aspect of the cutin spectrum. Moreover this method is solvent and time consuming.

Finally another possibility reported in literature to extract cutin is with an acid hydrolysis, as reported in J. J. Benitez, R. Garcia-Segura, A. Heredia, "Plant biopolyester cutin: a tough way to its chemical synthesis", Biochimica et Biophysica Acta 1674 (2004) 1-3. In this method cutin samples were obtained after hydrolysis of dewaxed cuticles in a 6 M HCl solution for 12 h at 105° C to remove polar hydrolysable components and then depolymerized in a 3% (w/v) sodium methoxide solution for 18 h at 100° C. After extraction of the monomers of tomato fruit cutin in an organic phase (diethyl ether), the solvent was evaporated to quantify and identify the cutin monomers by gas chromatography-mass spectrometry analysis.

The above method has been experimented, but the yield was very low and the extract showed a bad ability to form a new biolacquer.

WO2010093320 discloses a method for converting suberin and/or cutin containing plant parts into a suberin monomer containing mixture by alkali hydrolysis and isolating a fraction enriched in c/s-9,10-epoxy-18-hydroxyoctadecanoic acid together with a residual fraction containing, mainly more lipophilic hydroxyfatty acids, betulin, lupeol, and betulinic acid as major components.

### OBJECT OF THE INVENTION

The invention consists in a solubilization of waste tomato skin in alkaline solution, in fact the skin is for the most part (about 60-80%) solubilized by an alkaline solution and subjected to a thermal treatment at a temperature of 100°C for around 6 hours.

Subsequently, the raw bioresins, in solution as sodic resinates, are treated with acid to reduce pH and they are separated and cleaned of impurities, first by precipitation and then by centrifugation.

In this way a raw bioresin, partially depolimerized, is obtained in a pasty mass containing around 40% of water.

### BRIEF DESCRIPTION OF THE DRAWINGS

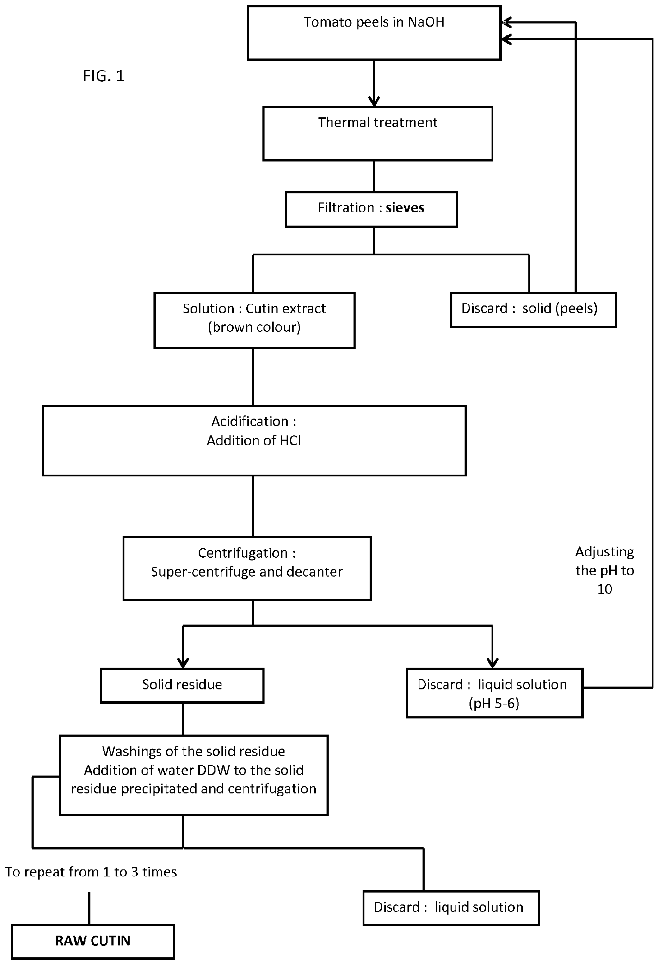

The invention is subsequently explained in more detail with reference to exemplary embodiments in conjunction with the drawings, in which the Figure 1 shows the extraction protocol of cutin, object of said invention.

### DETAILED DESCRIPTION

Cutin is extracted from waste tomato peels and precisely cutin is obtained in the extraction of a polyester polymer composed of a complex mixture of interesterified, long-chain 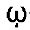 -hydroxy acid with typically a 16- or 18-carbon skeleton.

Some different kinds of peels can be utilized: desiccated peels, fresh frozen peels and peels from which was extracted lycopene.

At an initial stage of the claimed extraction method from wasted tomato peels, tomato skins and seeds are separated by flotation with flow water.

Then, the claimed process provides the following macro-steps:
- Thermal treatment of the tomatoes peels
- Filtration
- Acidification
- Centrifugation
- Drying phase (eventually)

So, the first step relates to the thermal treatment; tomato peels are subjected to a thermal treatment, preferably in an autoclave device, where the tomato peels are immerged in an alkaline solution (preferably NaOH) at 3% in weight, a concentration included between 0.5 M and 6 M (preferably 0.75 M); the temperature is included between 65°C and 130°C for a time more than 15 minutes and less than 6 hours (preferably around 2 hours); the ratio weight tomato peels/V NaOH can chances from 0.1 to 10.

After the above thermal treatment phase the solution is filtered so that solid residue can be separated by liquid; in this way the solid residue can be discarded or re-introduced in the process, while the liquid filtrate is kept. This liquid solution filtered is of brown colour.

Follow the acidification phase in which said filtrated liquid solution is added with an inorganic acid, and more precisely HCl with a concentration included between 12M and 6M, until the solution changes its colour and from brown it became ochre.

Preferably it is added around 10% (preferably 5%) HCl of the total volume and the pH of the solution after the color changing is comprises in the interval 5 - 6.

After the above acidification phase, the subsequent step is the centrifugation phase in which the solution is centrifuged at a range of 10000-14000 rpm for 15-20 minutes.

After the centrifugation the supernatant was discarded or reintroduced in the process for another extraction, but in the latter case only after the pH is being adjusted to the original value, preferably around 10.

The solid residue is kept and centrifuged with water DDW from 1 to 3 times in the same conditions; these further steps having the scope of washed/cleaned the solid residue.

In this way a raw bioresin, partially depolimerized, is obtained in a pasty mass containing around 25-80% of water.

A final stage could be applied: said final phase consisting of a drying phase for evaporating the water contained in the pasty mass.

By means of GC-MS analysis, the yield in 10,16 - dihydroxyhexadecanoic acid is included between 60-80%.

The yield of extraction has been included between 10 and 30% (around 15%).

To determine the percentage of water present in the extracted raw cutin it's necessary to heat the weighted sample of raw cutin (1 g) at 100°C for 4 hours in a specific sample pan, previously treated at 200°C for 30 minutes (passivation's treatment). The percentage of solid residue is calculated from the weight's difference between the initial weight of sample and the weight of sample after the thermal treatment.

## Claims

1. Method for extraction of a polyester polymer composed of a complex mixture of interesterified, long-chain 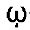 -hydroxy acid with typically a 16- or 18-carbon skeleton from the waste of tomato peels, **characterized in that** said method comprising the step of:
a. Thermal treatment of the tomatoes peels, in which tomato peels are immerged in an alkaline solution,
b. Filtration, for separating solid residue by liquid; then the filtrated liquid is kept for a step of
c. Acidification adding an inorganic acid until the solution changes its color so that the pH of the solution after the color changing is comprises in the interval 5 - 6
d. Centrifugation phase, in which the solution is centrifuged at a range of 10000-14000 rpm for 15-20 minutes; after the centrifugation the supernatant is discarded or reintroduced in the process for another extraction, while the solid residue is kept and centrifuged with water DDW from 1 to 3 times in the same conditions; these further steps having the scope of washed/cleaned the solid residue.

2. Method according to claim 1, **characterized in that** said thermal treatment in alkaline solution occurs with NaOH at 3% in weight, a concentration included between 0.5 M and 6 M and at a temperature included between 65°C and 130°C for a time more than 15 minutes and less than 6 hours.

3. Method according to claim 2, **characterized in that** said thermal treatment in alkaline solution provides a concentration of 0.75 M and at a temperature included between 65°C and 130°C for around 2 hours.

4. Method according to claim 1, **characterized in that** after filtration phase solid residue is re-introduced in the process.

5. Method according to claim 1, **characterized in that** said inorganic acid is Hydrogen chloride (HCl) with a concentration included between 12M and 6M, preferably 5% HCl of the total volume.

6. Method according to claim 1, **characterized in that** after centrifugation phase a supernatant is obtained and reintroduced in the process for another thermal treatment; before of this the PH of the supernatant is adjusted at the original value, around 10.

7. Method according to claim 1, **characterized in that** a final stage could be applied; said phase consisting of a drying phase of the pasty mass obtained with the method.

## Patentansprüche

1. Verfahren zur Extraktion eines Polyesterpolymers aus einem komplexen Gemisch bestehend aus langkettigen umgeesterte ω-Hydroxid-Säure, in der Regel mit einer Struktur von 16 oder 18 Kohlenstoff aus Tomatenschalenabfall, **dadurch gekennzeichnet, dass** das Verfahren die folgenden Operationen umfasst:
a. Wärmebehandlung von Tomatenschalen, bei der die Tomatenschalen in eine alkalische Lösung getaucht werden,
b. Filtrieren, um feste Rückstände durch einer Flüssigkeit abzutrennen, wobei die gefilterte Flüssigkeit dafür verwendet wird:
c. Ansäuern unter Zugabe einer anorganischen Säure, bis die Lösung ihre Farbe geändert hat, wobei der pH-Wert der Lösung nach der Farbänderung im Bereich von 5 bis 6 liegt
d. Zentrifugier-Phase, in der die Lösung 15 bis 20 Minuten bei einer Geschwindigkeit von 10.000 bis 14.000 U/min zentrifugiert wird; nach der Zentrifugier-Phase wird der Überstand zur weiteren Extraktion abgelassen oder wieder ins Prozess eingeführt, während der feste Rückstand 1 bis 3 Mal unter den gleichen Bedingungen aufbewahrt und mit DDW-Wasser zentrifugiert wird; diese weiteren Vorgänge haben den Zweck, den festen Rückstand zu waschen/reinigen.

2. Verfahren nach Anspruch 1, **dadurch gekennzeichnet, dass** die Wärmebehandlung in einer alkalischen Lösung mit 3 Gew.-% NaOH mit einer Konzentration zwischen 0,5 M und 6 M und einer Temperatur zwischen 65°C und 130°C für mehr als 15 Minuten und weniger als 6 Stunden erfolgt.

3. Verfahren nach Anspruch 2, **dadurch gekennzeichnet, dass** die Wärmebehandlung in einer alkalischen Lösung eine Konzentration von 0,75 M und bei einer Temperatur zwischen 65°C und 130°C für etwa 2 Stunden, liefert.

4. Verfahren nach Anspruch 1, **dadurch gekennzeichnet, dass** nach der Filtrations-Phase der feste Rückstand wieder ins Prozess eingeführt wird.

5. Verfahren nach Anspruch 1, **dadurch gekennzeichnet, dass** die anorganische Säure Chlorwasserstoff (HCl) mit einer Konzentration zwischen 12 M und 6 M, vorzugsweise 5% HCl des Gesamtvolumens, ist.

6. Verfahren nach Anspruch 1, **dadurch gekennzeichnet, dass** nach der Zentrifugier-Phase ein Überstand erhalten und zur weiteren Wärmebehandlung wieder ins Prozess eingeführt wird; davor wird der pH-Wert des Überstands auf den ursprünglichen Wert eingestellt, der etwa 10 entspricht.

7. Verfahren nach Anspruch 1, **dadurch gekennzeichnet, dass** eine erste Phase angewendet werden könnte: die Phase besteht in einem Vorgang des Trocknens der mit dem Verfahren erhaltenen pastösen Masse.

## Revendications

1. Procédé d'extraction d'un polymère polyester constitué d'un mélange complexe d'acide ω-hydroxyde à longue chaîne interestérifié, normalement de structure 16 ou 18 carbone à partir de déchets de pelure de tomate, **caractérisé en ce que** ledit procédé comprend les opérations suivantes:
à. Traitement thermique des pelures de tomates, dans lequel les pelures de tomates sont immergées dans une solution alcaline,
b. Filtrage pour séparer les résidus solides par un liquide, puis le liquide filtré est conservé pour une opération de:
c. Acidification avec addition d'un acide inorganique jusqu'à ce que la solution ait changé de couleur, le PH de la solution après le changement de couleur étant compris entre 5 et 6
d. PHase de centrifugation, dans laquelle la solution est centrifugée à une vitesse de 10.000 à 14.000 tr/min pendant 15 à 20 minutes; après centrifugation, le surnageant est déchargé ou réintroduit dans le procédé pour une extraction supplémentaire, tandis que le résidu solide est conservé et centrifugé avec de l'eau DDW de 1 à 3 fois dans les mêmes conditions; ces opérations supplémentaires ayant pour but de laver/nettoyer le résidu solide.

2. Procédé selon la revendication 1, **caractérisé en ce que** le traitement thermique en solution alcaline a lieu avec du NaOH à 3% en poids, avec une concentration comprise entre 0,5 M et 6 M et à une température comprise entre 65°C et 130°C pour plus de 15 minutes et moins de 6 heures.

3. Procédé selon la revendication 2, **caractérisé en ce que** ledit traitement thermique en solution alcaline fournit une concentration de 0,75 M à une température comprise entre 65°C et 130°C pendant environ 2 heures.

4. Procédé selon la revendication 1, **caractérisé en ce qu'**après l'étape de filtration, le résidu solide est réintroduit dans le procédé.

5. Procédé selon la revendication 1, **caractérisé en ce que** ledit acide inorganique est du chlorure d'hydrogène (HCl) avec une concentration comprise entre 12 M et 6 M, de préférence de 5% HCl du volume total.

6. Procédé selon la revendication 1, **caractérisé en ce qu'**après l'étape de centrifugation un surnageant est obtenu et réintroduit dans le procédé pour un autre traitement thermique; avant cela, le PH du surnageant est ajusté à la valeur d'origine, égale à environ 10.

7. Procédé selon la revendication 1, **caractérisé en ce qu'**une étape finale pourrait être appliquée: ladite opération consistant en une opération de séchage de la masse pâteuse obtenue avec le procédé.
